# EUROPÄISCHE PATENTSCHRIFT

(11) **EP 1 912 058 B1**
(45) Veröffentlichungstag und Bekanntmachung des Hinweises auf die Patenterteilung: **15.12.2010**
(21) Anmeldenummer: 06021584.5
(22) Anmeldetag: 14.10.2006
(51) Int. Cl.: G01N 21/27, G01N 21/47, G01N 21/86

(54) **Anordnung und Verfahren zum Erfassen und Auswerten optischer Signale**
Apparatus and method for detecting and evaluating optical signals
Dispositif et procédé pour la détection et l'évaluation des signaux optiques

(43) Veröffentlichungstag der Anmeldung: 16.04.2008
(73) Patentinhaber: Roche Diagnostics GmbH, 68305 Mannheim (DE); F. Hoffmann - La Roche AG, 4070 Basel 90265 (CH)
(72) Erfinder: Wehowski, Frederic, 68766 Hockenheim (DE); Rösicke, Bernd, 68305 Manheim (DE); Kalveram, Stefan, 68519 Viernheim (DE)
(74) Vertreter: Petirsch, Markus

(56) Entgegenhaltungen:
- DE-A1- 10 062 126
- DE-A1- 19 708 216
- DE-C1- 19 911 325
- JP-A- 58 225 345
- US-B1- 6 574 425
- US-B2- 6 562 625

## Beschreibung

Die Erfindung betrifft eine Anordnung zum Erfassen und Auswerten optischer Signale, um einen Analyten in einer Analyseflüssigkeit zu detektieren, mit einem Testträger, der eine optische Auswertezone aufweist. Die Anordnung umfasst eine Lichtquelle, um die optische Auswertezone auf dem Testträger zu beleuchten, eine Signalquelle, um ein erstes Ansteuersignal mit einer Frequenz F1 und einer Intensität I1 für die Lichtquelle zu erzeugen und einem Lichtsensor, um von dem Testfeld remittiertes Licht zu empfangen, zu verarbeiten und in ein Messsignal umzuwandeln. Einem ersten frequenzselektiven Verstärker wird das Messsignal sowie das erste Ansteuersignal der Signalquelle zugeführt. An dem Ausgang des Verstärkers wird ein erstes Ausgangssignal ausgegeben.

Weiter richtet sich die Erfindung auf ein Verfahren zum Erfassen und Auswerten optischer Signale, bei dem das von einem Lichtsensor empfangene Licht in ein Messsignal umgewandelt wird, dass einem frequenzselektiven Verstärker zugeführt wird.

Zur Bestimmung eines Analyten in einer Analyseflüssigkeit, insbesondere in Körperflüssigkeiten wie Blut zum Zwecke der medizinischen Prüfung, werden Analysesysteme eingesetzt, die mit photometrischen Signalen arbeiten und bei denen auf die Analysegeräte abgestimmte Testträger verwendet werden. Die Testträger (die auch als Analyseelemente bezeichnet werden) sind meist in Form von Teststreifen ausgebildet, In der Regel weisen sie ein oder mehrere Testfelder auf, die aus wenigstens einer Testschicht aufgebaut sind. Die Testschicht enthält ein oder mehrere Reagenzien. Beim Aufbringen einer Analyseflüssigkeit erfolgt eine chemische Reaktion der Inhaltsstoffe der Analyseflüssigkeit, die zu einer nachweisbaren Änderung des Testfelds, insbesondere zu einer Farbveränderung führt. Diese Veränderung wird mittels geeigneter Verfahren ausgewertet. Beispielsweise kann bei einer reflexionsphotometrischen Messung nach Ablauf der chemischen Reaktion aus dem diffusen Reflexionsvermögen des Testfelds auf die Konzentration eines zu bestimmenden Inhaltsstoffes der Analyseflüssigkeit geschlossen werden. In anderen Fällen kann man aus der zeitlichen Änderung der Reflektivität das gewünschte Ergebnis ableiten.

Es sind auch andere mit optischer Auswertung arbeitende Analysesysteme bekannt, insbesondere Systeme, deren Testträger als Kapillarteststreifen ausgebildet sind. Dabei befinden sich die Reagenzien in einem Kapillarkanal, der auch eine optische Messzone aufweist. Daneben sind auch Systeme mit optischen Referenzkanälen bekannt, wie auch Systeme, die auf Basis von Fluoreszenzmessungen arbeiten.

Die Erfindung richtet sich auf optische Analysesysteme beliebiger Konstruktion, soweit sie Testträger zur Aufnahme einer Analyseflüssigkeit mit einer optischen Auswertezone verwenden, in der mittels lichtoptischer Messmethoden eine optisch messbare Änderung als Maß für das gewünschte analytische Resultat detektiert wird. In erster Linie geht es um photometrische Verfahren, bei denen auf die optische Auswertezone gestrahltes Licht diffus reflektiert wird. Daneben ist die Erfindung aber auch für Fluoreszenzmessungen geeignet. Vielfach wird die optische Auswertezone auch als "Testfeld" bezeichnet. Nachfolgend wird auch dieser Begriff ohne Beschränkung der Allgemeinheit zur Bezeichnung der optischen Auswertezone verwendet.

Die Erfassung und Auswertung der optischen Signale stellt besonders hohe Anforderungen an die Genauigkeit, um die sehr geringen Messströme oder Spannungen mit einer hinreichenden Auflösung zu bestimmen und eine quantitative Analyse zu ermöglichen. Die Messungen werden von einer Vielzahl von Störquellen beeinflusst, zu denen die typischen Probleme beim Messen kleiner Signale gehören. Diese sind beispielsweise Verstärkerdrift oder überlagerte Gleich- oder Wechselspannungen, insbesondere niederfrequente Störspannungen, sowie Störströme unterschiedlicher Art.

Neben diesen Störungen bestehen optische Störungen durch Fremdlicht. Hierzu zählen konstante Fremdlichtanteile und modulierte Störungen insbesondere aus netzbetriebenen Fremdlichtquellen, die bei den typischen Netzfrequenzen und deren Harmonischen einwirken.

Es sind Analysegeräte mit Testträgern bekannt, die durch konstruktive Maßnahmen das Eintreten von Fremdlicht in dem Messbereich des Gerätes, insbesondere auf die optische Auswertezone reduzieren. Diese Geräte haben einen engen Kanal, in den der Testträger in das Gehäuseinnere eingeschoben wird. Eine andere Ausführungsform hat einen Deckel, der nach Einführung des Teststreifens und vor Beginn der Messung geschlossen werden muss. Diese konstruktiven Maßnahmen sind jedoch mit großen Nachteilen in der Benutzung verbunden. Beispielsweise kann bei einem zu engen Kanal die Analyseflüssigkeit während des Einschiebens verschmutzt werden. Außerdem verlängert sich die Messzeit bei diesen Geräten durch die Zeit, die die Analyseflüssigkeit benötigt, bis sie an der Messeinheit anliegt.

Neben der konstruktiven mechanischen Unterdrückung des Störlichts sind Systeme bekannt, bei denen eine elektronische Unterdrückung der Störungen durch Fremdlicht vorgenommen wird. Beispielsweise offenbart die US 6,574,425 B1 einen Reflektormeter, um Störlicht zu detektieren. Ein Photoempfänger gibt zwei phasenverschobene Ausgangssignale an einen Differentialverstärker weiter, dessen Ausgangssignal einem Detektor zugeführt wird. Der Detektor wird von derselben Signalquelle gesteuert, die auch die Lichtquelle ansteuert. Der Detektor erzeugt ein DC-Signal, dass der Farbe oder Farbänderung eines Testfelds entspricht. Die WO01/22871 A1 zeigt ein Beispiel eines optischen Glucose-Messsystems, bei dem ein frequenzselektiver Verstärker in Form eines Lock-In Amplifiers eingesetzt wird. Der Lock-In Amplifier wird zum einen mit dem Messsignal eines magnetooptischen Sensors gespeist. Zum anderen erhält er als Referenzsignal ein Signal eines externen Signalgenerators mit einer vorgegebenen Frequenz. Dadurch wird selektiv der Signalanteil des Messsignals verstärkt, dessen Frequenz dem Referenzsignal des Signalgenerators entspricht. Durch die Verwendung des Lock-In Verstärkers wird also eine Filterung für eine bestimmte Frequenz vorgenommen. Störlicht mit Frequenzen, die von der Frequenz des Referenzsignals verschieden sind, wird nicht oder nur stark gedämpft verstärkt und damit in der Messung nicht bzw. nur entsprechend reduziert berücksichtigt. Zur weiteren Verbesserung dieser Systeme wird vorgeschlagen, zwei Lock-In Verstärker in Reihe zu schalten. Das Ausgangssignal des ersten Lock-In Verstärkers wird also dem zweiten Lock-In Verstärker zugeführt. Dadurch wird das Signal-Rausch-Verhältnis des Sensors weiter verbessert. Weist eine Störung jedoch die Frequenz des Referenzsignals auf, wird auch die Störgröße verstärkt und ein falsches Meßergebnis ausgegeben. Das Auftreten der Störung und die Verfälschung des Messergebnisses kann von dem System nicht erkannt werden.

Die Verwendung von Lock-In Verstärker und die Kombination von mehreren Verstärkern, wie auch das kaskadierte Hintereinanderschalten von zwei Verstärkern ist beispielsweise aus der "Application Note AN1003, Low Level Optical Detection using Lock-In Amplifier Techniques" der Firma "AMETEK Signal Recovery" bekannt.

Der Einsatz von Lock-In Verstärkern ist beispielsweise in der Gasanalyse bekannt. In DE 10062126A1 wird offenbart, dass zwei Lock-In Verstärker zu Detektion einer Leckage eingesetzt werden können. DE 199 11 325 beschreibt die Verwendung von Lock-In Verstärkern bei der Gasanalyse, um einen akustooptisch durchstimmbaren Filter zu steuern.

Einen anderen Weg beschreiten die in der US 5,463,467 (DE 43 21 548 A1) und der US 4,553,848 (DE 3138879 A1) offenbarten Verfahren zum Erfassen und Auswerten analoger photometrischer Signale, bei denen das Testfeld eines Testträgers von einer in Hell-Dunkel-Phasen getakteten Lichtquelle bestrahlt wird. Das remittierte Licht wird während einer mehrere Hell-Dunkel-Phasen umfassenden Messperiode von einem Messempfänger aufgenommen und integriert. Eine effektive Stör- und Fremdlichtunterdrückung, die eine Messung auch ohne die bisher übliche Abschirmung gegen Umgebungslicht ermöglicht, wird durch eine unregelmäßige Abfolge der während der Messperiode integrierten Hell- und Dunkelphasen erzielt. Die Abfolge ist derart unregelmäßig, dass das fouriertransformierte Frequenzspektrum eine Vielzahl unterschiedlicher Frequenzen enthält, so dass jede einzelne Frequenz nur mit einem kleinen Bruchteil in das Messergebnis eingeht. Entsprechende Störfrequenzen können sich also auch nur mit einem Bruchteil-Fehlerbeitrag auf die Verfälschung des Messergebnisses auswirken.

Der Erfindung liegt die Aufgabe zugrunde, die Unterdrückung von Störsignalen beim Erfassen und Auswerten optischer Signale zur Bestimmung eines Analyten in einer Analyseflüssigkeit zu verbessern und die Zuverlässigkeit der Bestimmung zu erhöhen.

Gelöst wird die Aufgabe durch eine Anordnung mit den Merkmalen des Anspruchs 1 sowie durch ein Verfahren mit den Merkmalen des Anspruchs 12.

Die erfindungsgemäße Anordnung zum Erfassen und Auswerten optischer Signale mit einem Testträger, der ein Testfeld aufweist, umfasst eine Lichtquelle, um das Testfeld auf dem Testträger zu beleuchten, und einen Lichtsensor, um das von dem Testfeld remittierte Licht zu empfangen, zu verarbeiten und in ein Messsignal umzuwandeln. Ein erstes Ansteuersignal mit einer Frequenz F1 und einer Intensität I1 und ein zweites Ansteuersignal mit einer Frequenz F2 und einer Intensität I2 werden von je einer getrennten Signalquelle erzeugt. Die Anordnung umfasst eine Mischereinheit, um ein Lichtsteuersignal aus dem ersten Ansteuersignal und dem zweiten Ansteuersignal zu erzeugen, mit dem die Lichtquelle angesteuert wird.

Einem ersten frequenzselektiven Verstärker und einem zweiten frequenzselektiven Verstärker wird das von dem Lichtsensor empfangene Messsignal sowie das erste Ansteuersignal der ersten Signalquelle bzw. das zweite Ansteuersignal der zweiten Signalquelle zugeführt. An den Ausgängen der Verstärker wird ein erstes Ausgangssignal bzw. ein zweites Ausgangssignal ausgegeben. Beide werden in einer Auswerteeinheit verglichen. Aus dem Ergebnis des Vergleichs wird eine Information über eine Störung der Messung, insbesondere durch Fremdlicht, ermittelt.

Die Operation der Auswerteeinheit kann in der Praxis so ablaufen, dass das Ergebnis des Vergleichs der Ausgangssignale mit einem Soll-Vergleichsergebnis verglichen wird, das bei störungsfreier Funktion (und anderweitig gleichen Bedingungen) zu erwarten ist. Im einfachsten Fall sind die beiden Ausgangssignale bei störungsfreier Funktion gleich. Das Soll-Vergleichsergebnis ist dann die Übereinstimmung beider Signale. Es kann aber auch eine funktionale Beziehung zwischen den beiden Ausgangssignalen bestehen. In diesem Fall wird die funktionale Abhängigkeit in der Auswerteeinheit durch das Soll-Vergleichsergebnis berücksichtigt. Beispielsweise kann der Wert des ersten Ausgangssignals ein Vielfaches des Wertes des zweiten Ausgangssignals sein, insbesondere wenn die Intensität des ersten Ansteuersignals ein Vielfaches der Intensität des zweiten Ansteuersignals ist. Die funktionale Beziehung zwischen den Ausgangssignalen kann neben der Proportionalität auch durch eine andere Funktion gegeben sein.

Der in der Auswerteeinheit durchgeführte Vergleich muss sich nicht unmittelbar auf die Ausgangssignale beziehen, sondern sie können auch indirekt miteinander verglichen werden. Wichtige Beispiele sind der Vergleich referenzierter Ausgangssignale und von aus den Ausgangssignalen mittels Kalibrationsdaten ermittelten analytischen Ergebnissen. Beides wird noch näher erläutert.

Die auf der Grundlage des Vergleichs der Ausgangssignale gewonnene Information über eine Störung der Messung, insbesondere durch Fremdlicht, kann - abhängig von der Art des Ergebnisses ("störungsfrei" oder "gestört") und anderen Faktoren unterschiedlich genutzt und weiterverarbeitet werden:
- Wenn eine Störung vorliegt, kann diese Information insbesondere zur Erzeugung einer Fehlermeldung, beispielsweise als Klartext auf einem Display oder als sonstiges optisches oder akustisches Signal verarbeitet werden. Außerdem kann sie verwendet werden, um die weitere Verarbeitung der Ausgangssignale zu einem analytischen Resultat zu sperren.
- Die durch den Vergleich gewonnene Information kann aber auch als internes Steuerungssignal des Analysesystems weiterverarbeitet werden, durch das die Ausgabe eines analytischen Resultates, beispielsweise der gemessenen Glucosekonzentration, freigegeben wird, wenn das Ergebnis des Vergleichs dem Soll-Vergleichsergebnis entspricht.

Unabhängig von der Art der Weiterverarbeitung wird das aus der Vergleichsoperation resultierende, die Information über das Vorliegen einer Störung enthaltende Signal nachfolgend als "Auswertungssignal" bezeichnet.

Sinnvollerweise wird der frequenzselektive Verstärker möglichst so eingestellt, dass er nicht auf der Störfrequenz der Störung arbeitet. In der Regel ist die Frequenz der auftretenden Störfrequenzen jedoch nicht vollständig bekannt. Erfindungsgemäß wird eine parallele Messung durchgeführt, wobei mindestens zwei Messungen gleichzeitig vorzunehmen sind und bevorzugt in einer Anordnung realisiert werden. Die beiden Frequenzen F1 und F2 der Ansteuersignale sind in dem Lichtsteuersignal enthalten, das in der Mischereinheit aus dem ersten und zweiten Ansteuersignal erzeugt wird. Diese beiden Frequenzen F1 und F2 können gemäß der Erfindung wieder aus dem vom Lichtsensor ausgegebenen Messsignal extrahiert und herausgefiltert werden.

Die Lichtquelle wird mit dem aus der Mischung resultierende Lichtsteuersignal angesteuert und zwei frequenzselektive Verstärker werden zur Auswertung des Messsignals parallel geschaltet, die mit der Frequenz F1 bzw. F2 arbeiten, da sie mit dem ersten bzw. zweiten Ansteuersignal als Referenzsignal angesteuert werden. Es wird also praktisch die Wirkung von zwei parallel geschalteten Bandpassfiltern mit einem Filterband um die Frequenz F1 bzw. F2 erzielt.

Durch die Parallelschaltung der beiden frequenzselektiven Verstärker kann mittels eines einfachen Vergleichs erkannt werden, ob ein Störsignal dem Messsignal überlagert ist. Nur wenn eine Störung in beiden, den Frequenzen der Ansteuersignale entsprechenden Frequenzbereichen mit dem gleichen Intensitätsverhältnis einwirkt, das die Ansteuersignale aufweisen, besteht das Risiko, dass sie nicht erkannt wird. Dies ist sehr unwahrscheinlich. Da die Störungen in der Regel relativ schmalbandig sind, jedenfalls kein breites Frequenzspektrum mit gleicher Intensität abdecken, werden auftretende Störgrößen durch die Parallelschaltung von zwei frequenzselektiven Verstärkern zuverlässig erkannt.

Durch das zuverlässige Erkennen eines Fehlers wird die Messung deutlich sicherer. Insbesondere bei der Glucosebestimmung im Blut ist ein abgesichertes Ergebnis, das frei von Störeinflüssen ist, wichtig, da eine weiterführende Therapie für den Patienten auf dieser Messung beruht, z.B. die Insulindosierung.

Die Produktionskosten für einen selektiven Frequenzverstärker sind bei großen Stückzahlen gering. Die Gesamtkosten der Messanordnung steigen nur unwesentlich durch die Verwendung eines zweiten frequenzselektiven Verstärkers. Als frequenzselektive Verstärker werden bevorzugt Lock-In Verstärker eingesetzt. Diese haben die Funktion eines sehr scharfen frequenzselektiven Filters, da nur die Frequenzanteile des Messsignals verstärkt werden, die der Frequenz eines Referenzsignals entsprechen.

Bei mehrfachem oder permanentem Auftreten von Störungen können die Ansteuerfrequenzen F1 und F2 der Ansteuersignale in solche Bereiche verschoben werden, in denen keine Störungen auftreten. Beispielsweise werden die Frequenzen F1 und F2 der Ansteuersignale beim Auftreten einer 50/60 Hertz-Störspannung so gewählt, dass sie ungleich 50/60 Hertz oder eines Vielfachen davon sind.

Der Begriff Signalquelle ist im Rahmen dieser Erfindung sehr allgemein zu verstehen. Im einfachsten Fall ist die Signalquelle ein Signalgenerator, mit dem ein Ansteuersignal, beispielsweise ein Sinussignal, ein Trapezsignal oder ein Rechtecksignal, erzeugt wird. Die Signalquelle kann allerdings auch zwei oder mehrere Signalgeneratoren umfassen, so dass in einer Signalquelle gleichzeitig mehrere Ansteuersignale generiert werden können.

Vorteilhafterweise ist das erste Ansteuersignal ein Rechtecksignal mit einer Grundfrequenz und das zweite Ansteuersignal eine Harmonische des ersten Ansteuersignals, dessen Frequenz ein Vielfaches der Grundfrequenz ist. Die Intensität 12 des zweiten Ansteuersignals ist bevorzugt von der Intensität des ersten Ansteuersignals verschieden. Das erste und zweite Ansteuersignal werden von einer ersten und einer zweiten Signalquelle erzeugt . Die Frequenz des zweiten Ansteuersignals ist dabei vorteilhaft ein ungradzahliges Vielfaches der Grundfrequenz. Bevorzugterweise wird die dritte Harmonische des Rechtecksignals verwendet, deren Frequenz dem dreifachen der Grundfrequenz entspricht. Die Intensität dieser Harmonischen ist etwa ein Drittel der Intensität der Grundfrequenz, d.h. die Intensität I2 ist ein Drittel der Intensität I1.

Es kann ein Signalgenerator, der nur ein Rechtecksignal erzeugt, als Signalquelle für zwei Frequenzen bzw. zwei Ansteuersignale mit unterschiedlichen Frequenzen (die Vielfache voneinander sind) verwendet werden. Selbstverständlich eignen sich auch die fünfte und siebte Harmonische, wobei die Intensität mit dem Grad der Harmonischen (Ordnungszahl) abnimmt. Insbesondere können zusätzlich die höheren Harmonischen (z.B. fünfte und siebte Harmonische) generiert werden, wenn mehrere Ansteuersignale erzeugt werden sollen, da mehrere frequenzselektive Verstärker zur Detektion der remittierten Lichtsignale in der Anordnung parallel geschaltet sind.

Als vorteilhaft hat sich eine Ausführungsform erwiesen, bei der in der Auswerteeinheit das erste Ausgangssignal mit einem ersten Referenzsignal ins Verhältnis gesetzt wird, wodurch ein erstes referenziertes Ausgangssignal entsteht. Ebenso wird mit dem zweiten Ausgangssignal verfahren, dass mit einem zweiten Referenzsignal so ins Verhältnis gesetzt wird, dass ein zweites referenziertes Ausgangssignal erzeugt wird. Die beiden referenzierten Ausgangssignale werden dann miteinander in Beziehung gesetzt, beispielsweise durch Verhältnisbildung. Durch die Verwendung eines Referenzsignals, mit dem das Ausgangssignal ins Verhältnis gesetzt wird, werden Schwankungen im Messsystem kompensiert. Dies ist insbesondere dann der Fall, wenn das Referenzssignal dem Ansteuersignal, das mit der Signalquelle erzeugt wird, entspricht. Schwankungen oder Abweichungen des Ansteuersignals werden dann herausgerechnet. Die Referenzsignale können auch durch eine "Leermessung" an einem leeren Testträger erzeugt werden, auf dem keine Analyseflüssigkeit aufgebracht ist.

Vorzugsweise sind die frequenzselektiven Verstärker in einem elektronischen Baustein implementiert. Beispielsweise können hier ASICs verwendet werden. Hierdurch wird die Herstellung einer Detektions- und Auswerteeinrichtung für photometrische Signale sehr kostengünstig. Auch ist es möglich, die frequenzselektiven Verstärker in Form von Software zu implementieren, die vorzugsweise in dem selben elektronischen Baustein abläuft. In diesem Fall, wenn das Signal nicht hardwaremäßig verstärkt wird, ist ein A/D-Wandler einzusetzen, der das Analogsignal in ein digitales Signal wandelt. Es kann dann durch eine Software multipliziert werden. Auf diese Weise entstehen durch einen zusätzlichen Lock-In Verstärker keine Zusatzkosten, da keine zusätzliche Hardware implementiert werden muss. Die Herstellungskosten werden reduziert. Eine Miniaturisierung der Anordnung wird möglich.

In einer bevorzugten Ausführungsform sind ein drittes Ansteuersignal und ein dritter frequenzselektiver Verstärker vorgesehen. Das dritte Ansteuersignal hat eine Frequenz F3 und eine Intensität 13. Das Lichtsteuersignal zum Ansteuern der Lichtquelle wird dann aus dem ersten, dem zweiten und dem dritten Ansteuersignal erzeugt. Dem dritten frequenzselektiven Verstärker, der den beiden anderen parallelgeschaltet ist, wird ebenfalls das Messsignal des Lichtsensors sowie das dritte Ansteuersignal zugeführt. Am Ausgang des dritten frequenzselektiven Verstärkers wird ein drittes Ausgangssignal ausgegeben, das neben dem ersten Ausgangssignal und dem zweiten Ausgangssignal der Auswerteeinheit zugeführt und mit diesen verglichen wird. Bei einer Abweichung eines Ausgangssignals wird die resultierende Störungsinformation bevorzugt dazu verwendet, dass das abweichende Ausgangssignal nicht berücksichtigt wird.

Beim Auftreten eines Störsignals, also dem Erkennen eines Fehlers, weicht ein Ausgangssignal von den beiden anderen ab. Die beiden anderen Ausgangssignale sind gleich. In diesem Fall ist also bekannt, dass das abweichende Ausgangssignal von einer Störgröße überlagert ist. Die beiden gleichen Ausgangssignale stellen das "richtige", also fehlerfreie, Messergebnis dar. Hierdurch lässt sich nicht nur eine Aussage darüber treffen, ob ein Fehler aufgetreten ist, sondern die beiden anderen Ausgangssignale können weiter verarbeitet werden, um das gewünschte analytische Resultat (Konzentration des Analyten in der Analyseflüssigkeit) zu ermitteln. Selbstverständlich lässt sich dies auf Ausführungsformen mit mehreren selektiven Verstärkern, z.B. mit N Verstärkern (N > 2, N ganzzahlig) ausweiten. In diesem Fall sind dann, wenn eine Störung nur auf die Frequenz eines Ansteuersignals fällt, die anderen N-1 Ausgangssignale gleich. Hierbei wird die sogenannte "Majoritätslogik" angewandt.

Die Erfindung wird nachfolgend anhand eines Ausführungsbeispiels unter Bezugnahme auf die beigefügte Zeichnung näher erläutert. Die darin dargestellten Besonderheiten können einzeln oder in Kombination verwendet werden, um bevorzugte Ausgestaltungen zu schaffen.

Es zeigt:

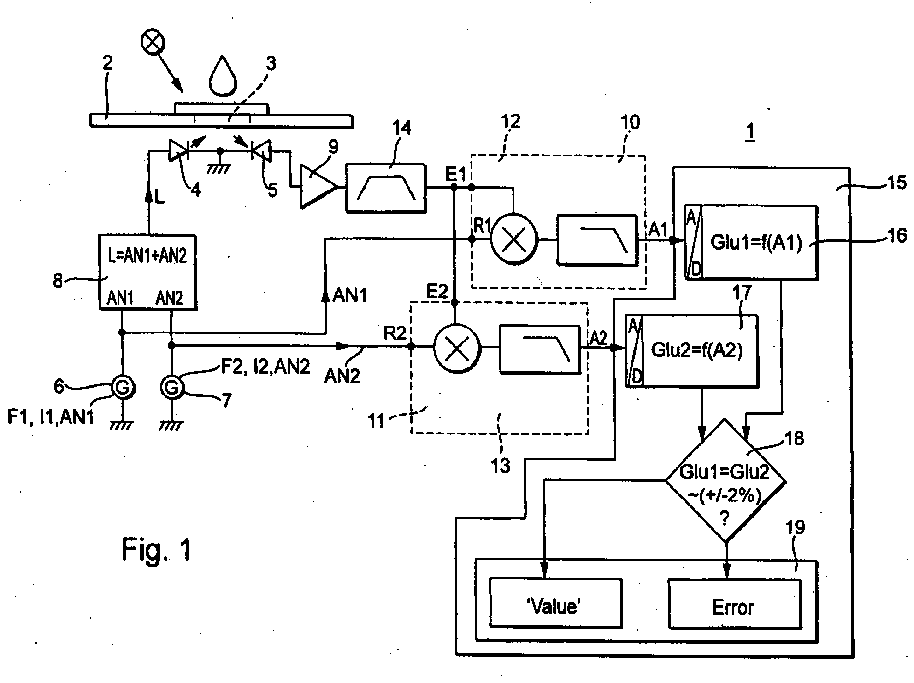

- Fig. 1: ein Prinzipschaltbild eines Ausführungsbeispiels einer erfindungsgemäßen Anordnung zum Erfassen und Auswerten photometrischer Signale.

Figur 1 zeigt ein Blutzuckermessgerät als Analysegerät 1 zur Erfassung eines Analyten. Mittels des Blutzuckermessgeräts wird der Glucosegehalt in einer Probenflüssigkeit, insbesondere in Blut, gemessen. Ein Testträger 2 ist als Teststreifen ausgebildet, auf dem Blut als Analyseflüssigkeit aufgegeben wird, das den zu untersuchenden Analyten, Glucose enthält. Ein Testfeld 3 des Testträgers 2 wird von einer Lichtquelle 4 des Analysegerätes 1 beleuchtet. Die Lichtquelle 4 ist eine lichtemittierende Diode. Selbstverständlich können auch mehrere beliebige Lichtquellen vorhanden sein, die parallel oder seriell verschalten sein können. Das von dem Testfeld 3 abgestrahlte Licht wird von einem Lichtsensor 5 empfangen, der als Photodiode ausgebildet ist.

Die Lichtquelle 4 wird von einer ersten Signalquelle 6 und einer zweiten Signalquelle 7 angesteuert. Die beiden Signalquellen 6,7 erzeugen jeweils ein Signal mit einer festen Frequenz und vorgegebenen Intensität. Die erste Signalquelle 6 erzeugt ein Ansteuersignal AN1 mit der Frequenz F1 und der Intensität I1. Die zweite Signalquelle 7 erzeugt ein Ansteuersignal AN2 mit der Frequenz F2 und der Intensität I2. Die jeweiligen Ansteuersignale AN1,AN2 der beiden Signalquellen 6,7 werden einer Mischereinheit 8 zugeführt, in der ein Lichtsteuersignal L erzeugt wird. Das Lichtsteuersignal L wird aus dem ersten Ansteuersignal AN1 der ersten Signalquelle 6 und dem zweiten Ansteuersignal AN2 der zweiten Signalquelle 7 gebildet, beispielsweise durch Mischung. Insbesondere können in der Mischereinheit 8 das erste Ansteuersignal AN1 und das zweite Ansteuersignal AN2 miteinander addiert werden. Das Lichtsteuersignal L wird der Lichtquelle 4 zugeführt, um sie anzusteuern.

In dem von dem Testfeld 3 diffus reflektierten Lichtanteil, der von der Lichtquelle 4 auf das Testfeld 3 gestrahlt und von dem Lichtsensor 5 empfangen wird, sind die Frequenzanteile des Lichtsteuersignals L, also des ersten Ansteuersignals AN1 und des zweiten Ansteuersignals AN2 enthalten. Das von dem Lichtsensor 5 aufgenommene Lichtsignal wird in einen Photostrom umgewandelt. Der Photostrom wird in einem Vorverstärker 9 in eine Spannung umgewandelt und verstärkt. Das Ausgangssignal des Vorverstärkers 9 wird einem ersten frequenzselektiven Verstärker 10 und einem zweiten frequenzselektiven Verstärker 11 parallel zugeführt. Die frequenzselektiven Verstärker 10,11 sind als Lock-In Amplifier 12 bzw. 13 ausgebildet.

Bevorzugt ist zwischen dem Lichtsensor 5 und den frequenzselektiven Verstärkern 10,11 ein Filter vorgesehen, der vorzugsweise als Bandpassfilter 14 ausgebildet ist. Der Bandpassfilter 14 ist insbesondere zwischen dem Vorverstärker 9 und den frequenzselektiven Verstärkern 10 bzw. 11 angeordnet. Der Bandpassfilter 14 begrenzt das Ausgangssignal des Vorverstärkers 9 auf einen Frequenzbereich, in dem die Frequenzen F1,F2 des ersten Ansteuersignals AN1 und des zweiten Ansteuersignals AN2 liegen. Vorzugsweise ist der Bandpassfilter 14 in dem Vorverstärker 9 integriert. Alternativ kann in dem Vorverstärker 9 auch ein Tiefpassfilter integriert sein. Dann kann der nachfolgende Bandpassfilter 14 durch einen Hochpassfilter ersetzt werden. Insgesamt ist jedenfalls ein Bandpass vor den Lock-In Verstärkern 12,13 geschaltet.

Das gefilterte Signal des Vorverstärkers 9 wird dem Eingang E1 des ersten Lock-In Amplifiers 12 und dem Eingang E2 des zweiten Lock-In Amplifiers 13 zugeführt. An einem Referenzeingang R1 des Lock-In Amplifiers 12 liegt das Ansteuersignal AN1 der ersten Signalquelle 6 an. An einem Referenzeingang R2 des zweiten Lock-In Amplifiers 13 liegt das zweite Ansteuersignal AN2 der zweiten Signalquelle 7 an.

Die Ausgangssignale A1,A2 werden einer Auswerteeinheit 15 zugeführt, in der sie verglichen gesetzt werden. An der Auswerteeinheit 15 wird ein entsprechendes Auswertungssignal ausgegeben, das beispielsweise auf einem Display angezeigt oder als Datenwert oder Signal an ein Verarbeitungsgerät (z.B. einen Computer) weitergegeben wird.

Vorteilhafterweise hat das Analysegerät 1 genau zwei frequenzselektive Verstärker 10,11, wie in Figur 1 gezeigt. In diesem Fall wird bei einer Abweichung der Ausgangssignale A1,A2 des ersten Verstärkers 10 und des zweiten Verstärkers 11 durch direkten Vergleich erkannt, dass die durchgeführte Messung fehlerhaft ist. Wenn die Intensitäten I1,I2 der beiden Ansteuersignale AN1,AN2 der ersten Signalquelle 6 und der zweiten Signalquelle 7 identisch sind, müssen auch die Ausgangssignale A1,A2 der Lock-In Amplifier 12,13 identisch sein.

Bevorzugt sind die Intensitäten der jeweiligen Ansteuersignale der Signalquelle verschieden. Die Intensität I1 des Ansteuersignals AN1 der ersten Signalquelle 6 und die Intensität I2 des Ansteuersignals AN2 der zweiten Signalquelle 7 weisen dann ein bestimmtes Verhältnis auf. Die beiden Ausgangssignale der Lock-In Amplifier 12,13 müssen ebenfalls das gleiche Verhältnis (Soll-Vergleichsergebnis) zueinander haben. Um dies zu prüfen, werden sie in Beziehung gesetzt. Ist das gleiche Verhältnis nicht gegeben, so wird eine Störung der Messung, beispielsweise durch Streulicht oder Umgebungslicht, erkannt.

Bevorzugt wird, jeweils mittels Kalibrierdaten, in der Auswerteeinheit 15 aus dem ersten Ausgangssignal A1 und aus dem zweiten Ausgangssignal A2 ein erstes und ein zweites analytisches Resultat abgeleitet. Die beiden analytischen Resultate sind Konzentrationswerte des Analyten, der in der Analyseflüssigkeit auf dem Testfeld 3 des Testträgers 2 enthalten ist. Dazu schließt die Auswerteeinheit 15 eine Analyseeinheit 16 und eine Analyseeinheit 17 ein. Zur Bildung der analytischen Resultate sind in Analyseeinheiten 16,17 die Kalibrationsdaten zum Beispiel als mathematische Funktion hinterlegt, die die Beziehung zwischen dem Ausgangssignal und analytischem Resultat widerspiegeln. Die Analyseeinheiten 16,17 weisen an ihrem Eingang jeweils einen Analog-Digital-Wandler (A/D-Wandler) auf. Die an den Eingängen der Analyseeinheiten 16,17 anliegenden Ausgangssignale A1 bzw. A2 der Lock-In Amplifier 12,13 werden in digitale Signale umgewandt, so dass im Weiteren in der Auswerteeinheit 15 digitale Signale verarbeitet werden können. Wenn die beiden Lock-In Amplifier 12,13 in Form von Software realisiert sind, kann auf die A/D-Wandler an den Eingängen der Analyseeinheiten 16,17 verzichtet werden. Dann ist jedoch ein A/D-Wandler direkt am Ausgang des Bandpassfilters 14 vorgesehen. Das Ausgangssignal des Bandpassfilters 14 wird dann digitalisiert, so dass an den Eingängen E1,E2 der Lock-In Amplifier 12,13 jeweils digitale Signale vorliegen. In diesem Fall muss nur ein A/D-Wandler vorgesehen sein, im Gegensatz zu zweien, wenn die Lock-In Amplifier 12,13 in Form von Hardwarekomponenten realisiert sind.

Wenn das Analysegerät 1 wie hier ein Blutzuckermessgerät ist und der zu bestimmende Analyt der Glucosegehalt im Blut, sind die analytischen Resultate, die mit der Analyseeinheit 16 bzw. 17 ermittelt werden, Werte des Glucosegehaltes im Blut. Beispielsweise kann eine Kalibrierung des Systems derart vorgenommen werden, dass eine Flüssigkeit mit einem bekannten Glucosegehalt auf den Teststreifen aufgebracht wird und die Ausgangssignale A1,A2 der Lock-In Amplifier 12,13 ermittelt werden. Dieser Wert wird dann als Kalibrationswert verwendet. Dies kann auch in einem Produktionsprozessschritt erfolgen und wird dann als Funktion auf dem Teststreifen gespeichert. Diese Information wird dann in den Analyseeinheiten 16,17 hinterlegt bzw. implementiert. Die Kalibrierung wird mit unterschiedlichen Glucosekonzentrationen und bei unterschiedlichen Frequenzen vorgenommen, wobei eine Vielzahl von Kalibrationsdaten gewonnen werden. Im einfachsten Fall werden die Daten in einer Tabelle hinterlegt, auf die die Analyseeinheiten 16,17 zugreifen können. Zwischenwerte werden durch Interpolation ermittelt. Je umfangreicher die Kalibrierdatenbank ist, desto genauer kann aus den Ausgangssignalen A1,A2 der Lock-In Amplifier 12,13 auf den in der Analyseflüssigkeit enthaltenen Glucosewert geschlossen werden.

In einem Vergleicher 18 werden die analytischen Resultate der Analyseeinheiten 16,17 miteinander verglichen. Das Ergebnis des Vergleichs wird dem Benutzer des Analysegeräts 1 optisch dargestellt, beispielsweise in einem Display 19. Wenn analytische Resultate verglichen werden, entspricht der Soll-Vergleichswert immer der Übereinstimmung der Resultate. Unterschiede des Ausgangssignals der Lock-In Verstärker werden durch die Kalibration ausgeglichen. Sind die beiden analytischen Resultate gleich, so wird im Display 19 der gemessene Glucosewert angezeigt; andernfalls wird eine Fehlermeldung ausgegeben. Dies kann in Form eines Textes, eines optischen Signals oder einer akustischen Ausgabe geschehen.

Es hat sich als vorteilhaft erwiesen, die Ausgangssignale der frequenzselektiven Verstärker nicht als Rohdaten (raw data) unmittelbar zu vergleichen, sondern dadurch in Beziehung zueinander zu setzen, dass man aus diesen Rohdaten die analytischen Resultate ermittelt und miteinander vergleicht. Hierdurch können Fehler in der Elektronik bei der Auswertung der Ausgangssignale A1,A2 der Lock-In Amplifier 12,13 mitberücksichtigt werden. Insbesondere bei einer Realisierung des Analysegeräts 1 mittels Mikroprozessoren werden Fehler, die auf einen Defekt der Mikroprozessoren beruhen, erkannt. Obwohl solche Fehler sehr selten sind, können sie auf einfache Weise ausgeschlossen werden, wenn die analytischen Resultate und nicht die Ausgangssignale A1,A2 der Lock-In Amplifier 12,13 miteinander verglichen werden.

Die Verwendung eines weiteren Lock-In Amplifiers und dementsprechend eines weiteren Ansteuersignals führt dazu, dass nicht nur ein Störeinfluss auf die Messung erkannt wird, sondern dass trotz der Störung ein richtiges Analyseergebnis ausgegeben werden kann. Die Wahrscheinlichkeit, dass eine Störung bei zwei oder mehr Frequenzen gleichzeitig und in gleicher Weise die Messung beeinträchtigt, ist sehr klein. Durch die Hinzunahme weiterer Lock-In Verstärker wird diese Wahrscheinlichkeit noch weiter vermindert. Insbesondere, wenn die Lock-In Amplifier softwareimplementiert sind, ist der konstruktive Aufwand so gering, dass auch mit mehreren Lock-In Verstärkern gearbeitet werden kann, um die Sicherheit des Ergebnisses weiter zu erhöhen.

## Patentansprüche

1. Anordnung ausgebildet zum Erfassen und Auswerten optischer Signale, um einen Analyten in einer Analyseflüssigkeit zu detektieren, umfassend:
- einen Testträger (2) zur Aufnahme einer Analyseflüssigkeit, der eine optische Auswertezone (3) aufweist,
- eine Lichtquelle (4), um die optische Auswertezone (3) des Testträgers (2) zu beleuchten;
- eine erste Signalquelle (6), ausgebildet, um ein erstes Ansteuersignal (AN 1) für die Lichtquelle (4) mit einer Frequenz F1 und einer Intensität I1 zu erzeugen,
- einen Lichtsensor (5), ausgebildet, um von der optischen Auswertezone (3) remittiertes Licht zu empfangen, zu verarbeiten und in ein Messsignal umzuwandeln;
- einen ersten frequenzselektiven Verstärker (10), dem das Messsignal sowie das erste Ansteuersignal (AN1) der ersten Signalquelle (6) zugeführt wird und an dessen Ausgang ein erstes Ausgangssignal (A1) ausgegeben wird,
**gekennzeichnet durch**
- eine zweite Signalquelle (7), ausgebildet, um ein zweites Ansteuersignal (AN2) mit einer Frequenz (F2) und einer Intensität (12) zu erzeugen,
- eine Mischereinheit (8), ausgebildet, um aus dem ersten Ansteuersignal (AN1) und dem zweiten Ansteuersignal (AN2) ein Lichtsteuersignal (2) zu erzeugen, mit dem die Lichtquelle (4) angesteuert wird,
- einen zweiten frequenzselektiven Verstärker (11), dem das Messsignal sowie das zweite Ansteuersignal (AN2) der zweiten Signalquelle (7) zugeführt wird und an dessen Ausgang ein zweites Ausgangssignal (A2) ausgegeben wird,
- eine Auswerteeinheit (15), der das erste Ausgangssignal (A1) und das zweite Ausgangssignal (A2) zugeführt werden, und die derart ausgebildet ist, dass die beiden Ausgangssignale (A1,A2) verglichen und aus dem Ergebnis des Vergleichs eine Information über eine Störung der Messung **durch** Fremdlicht ermittelt wird.

2. Anordnung nach Anspruch 1, **gekennzeichnet durch**
- eine dritte Signalquelle, um ein drittes Ansteuersignal mit einer Frequenz F3 und einer Intensität I3 zu erzeugen,
- wobei das Lichtsteuersignal (2) aus dem ersten Ansteuersignal (AN1), dem zweiten Ansteuersignal (AN2) und dem dritten Ansteuersignal erzeugt wird,
- einem dritten frequenzselektiven Verstärker, dem das Messsignal sowie das dritte Ansteuersignal der dritten Signalquelle zugeführt wird und an dessen Ausgang ein drittes Ausgangssignal ausgegeben wird,
- der Auswerteeinheit das dritte Ausgangssignal zugeführt wird und das erste Ausgangssignal (A1), das zweite Ausgangssignal (A2) und das dritte Ausgangssignal vergleicht.

3. Anordnung nach Anspruch 1, **dadurch gekennzeichnet, dass** genau zwei frequenzselektive Verstärker (10,11) vorgesehen sind und bei Abweichung der beiden Ausgangssignale (A1,A2) von einem Soll-Vergleichsergebnis die durchgeführte Messung als fehlerhaft erkannt wird.

4. Anordnung nach einem der vorhergehenden Ansprüche, **dadurch gekennzeichnet, dass** die Signalquellen (6,7) derart ausgebildet sind, **dass** die Intensitäten (I1,I2) des ersten und des zweiten Ansteuersignals (AN1,AN2) verschieden sind.

5. Anordnung nach einem der vorhergehenden Ansprüche, **dadurch gekennzeichnet, dass** die Signalquelle (6) derart ausgebildet ist, **dass** das erste Ansteuersignal (AN1) ein Rechtecksignal mit einer Grundfrequenz ist und die Signalquelle (7) derart ausgebildet ist, dass das zweite Ansteuersignal (AN2) eine Harmonische des ersten Ansteuersignals (AN1) ist, wobei die Frequenz (F2) des zweiten Ansteuersignals (AN2) ein Vielfaches der Grundfrequenz, vorzugsweise das Dreifache der Grundfrequenz ist, und die Intensität (I2) des zweiten Ansteuersignals von der Intensität (I1) des ersten Ansteuersignals verschieden ist

6. Anordnung nach einem der vorhergehenden Ansprüche, **dadurch gekennzeichnet, dass** die Auswerteeinheit (15) derart ausgebildet ist, dass .
- das erste Ausgangssignal (A1) mit einem ersten Referenzsignal ins Verhältnis gesetzt wird, wodurch ein erstes referenziertes Ausgangssignal erzeugt wird,
- das zweite Ausgangssignal (A2) mit einem zweiten Referenzsignal ins Verhältnis gesetz wird, wodurch ein zweites referenziertes Ausgangssignal erzeugt wird, und
- das erste referenzierte Ausgangssignal mit dem zweiten referenzierten Ausgangssignal verglichen wird.

7. Anordnung nach einem der Ansprüche 1 bis 4, **dadurch gekennzeichnet, dass**
- in der Auswerteeinheit (15) aus dem ersten Ausgangssignal (A1) mittels erster Kalibrationsdaten ein erstes analytisches Resultat abgeleitet wird,
- in der Auswerteeinheit (15) aus dem zweiten Ausgangssignal (A2) mittels zweiter Kalibrationsdaten ein zweites analytisches Resultat abgeleitet wird, und
- das erste und das zweite analytische Resultat verglichen werden, um die Information über eine Störung der Messung zu gewinnen.

8. Anordnung nach einem der vorhergehenden Ansprüche, **dadurch gekennzeichnet, dass** zwischen dem Lichtsensor (5) und dem frequenzselektiven Verstärker (10,11) ein Filter, vorzugsweise ein Bandpass-Filter (14), angeordnet ist.

9. Anordnung nach einem der vorhergehenden Ansprüche, **dadurch gekennzeichnet, dass** die frequenzselektiven Verstärker (10,11) in Form von Software in einem elektronischen Baustein implementiert sind.

10. Anordnung nach Anspruch 9, **dadurch gekennzeichnet, dass** die frequenzselektiven Verstärker (10,11) in dem selben elektronischen Baustein implementiert sind.

11. Verfahren zum Erfassen und Auswerten photometrischer Signale, um einen Analyten in einer Analyseflüssigkeit zu detektieren, die auf ein Testfeld (3) eines Testträgers (2) aufgebracht ist,
bei dem das Testfeld (3) von einer gespeisten Lichtquelle (4) beleuchtet wird und das von dem Testfeld (3) remittierte Licht von einem Lichtsensor (5) empfangen, verarbeitet und in ein Messsignal umgewandelt wird, das einem ersten frequenzselektiven Verstärker (10) zugeführt wird,
**gekennzeichnet durch** die folgenden Schritte:
- Erzeugen eines ersten Ansteuersignals (AN1) mit einer Frequenz F1 und einer Intensität I1 in einer ersten Signalquelle (6),
- Erzeugen eines zweiten Ansteuersignals (AN2) mit einer Frequenz F2 und einer Intensität I2 in einer zweiten Signalquelle (7),
- Generieren eines Lichtsteuersignals aus dem ersten Ansteuersignal (AN1) und dem zweiten Ansteuersignal (AN2) zum Ansteuern der Lichtquelle (4),
- Zuführen des von dem Lichtsensor (5) empfangenen Messsignals an den ersten frequenzselektiven Verstärker (10) und an einen zweiten frequenzselektiven Verstärker (11),
- Zuführen des ersten Ansteuersignals (AN1) an den ersten frequenzselektiven Verstärker (10) und Zuführen des zweiten Ansteuersignals (AN2) an den zweiten frequenzselektiven Verstärker (11),
- Zuführen eines ersten Ausgangssignals (A1) des ersten frequenzselektiven Verstärkers (10) und eines zweiten Ausgangssignals (A2) des zweiten frequenzselektiven Verstärkers (11) an eine Auswerteeinheit (15),
- Vergleichen des ersten Ausgangssignals (A1) mit dem zweiten Ausgangssignal (A2) in der Auswerteeinheit (15), und
- Ermitteln einer Information über eine Störung der Messung **durch** Fremdlicht aus dem Ergebnis des Vergleichs.

## Claims

1. A system adapted for measuring and evaluating optical signals for detecting an analyte in an analysis liquid, the system comprising:
- a test carrier (2) for receiving a analysis liquid, which has an optical evaluation zone (3), the system;
- a light source (4) for illuminating the optical evaluation zone (3) of the test carrier (2);
- a first signal source (6) adapted for generating a first control signal (AN1) for the light source (4) having a frequency (F1) and an intensity (I1),
- a light sensor (5) adapted for receiving light remitted from the optical evaluation zone (3), and for converting it into a measuring signal;
- a first frequency-selective amplifier (10), to which the measuring signal and the first control signal (AN1) of the first signal source (6) are fed, and which has an output at which a first output signal (A1) is output,
**characterized in that**
- a second signal source (7) adapted for generating a second control signal (AN2) having a frequency (F2) and an intensity (I2),
- a mixer unit (8) adapted for generating, from the first control signal (AN1) and the second control signal (AN2), a light control signal (2) with which the light source (4) is controlled,
- a second frequency-selective amplifier (11), to which the measuring signal and the second control signal (AN2) of the second signal source (7) are fed and which has an output at which a second output signal (A2) is output,
- an evaluation unit (15), to which the first output signal (A1) and the second output signal (A2) are fed, and which is adapted such that the two output signals (A1, A2) are compared and an information about interference of the measurement by external light is determined from the result of the comparison.

2. System according to Claim 1, **characterized by**
- a third signal source for generating a third control signal having a frequency (F3) and an intensity (I3),
- wherein the light control signal (2) is generated from the first control signal (AN1), the second control signal (AN2), and the third control signal,
- a third frequency-selective amplifier, to which the measuring signal and the third control signal of the third signal source are fed and which has an output at which a third output signal is output,
- the third output signal is fed to the evaluation unit and the first output signal (A1), the second output signal (A2) and the third output signal are compared.

3. System according to Claim 1, **characterized in that** precisely two frequency-selective amplifiers (10, 11) are provided and in the event of deviation of the two output signals (A1, A2) from a predefined comparison result, the measurement performed is recognized as faulty.

4. System according to any one of the preceding claims, **characterized in that** the signal sources (6, 7) are adapted such that the intensities (I1, I2) of the first and second control signals (AN1, AN2) are different.

5. System according to any one of the preceding claims, **characterized in that** the signal source (6) is adapted such that the first control signal (AN1) is a square-wave signal having a fundamental frequency and the signal source (7) is adapted such that the second control signal (AN2) is a harmonic of the first control signal (AN1), wherein the frequency (F2) of the second control signal (AN2) is a multiple of the fundamental frequency, preferably three times the fundamental frequency, and the intensity (I2) of the second control signal is different from the intensity (I1) of the first control signal.

6. System according to any one of the preceding claims, **characterized in that**, the evaluation unit (15) is adapted such that,
- a ratio is formed of the first output signal (A1) and a first reference signal whereby a first referenced output signal is generated,
- a ratio is formed of the second output signal (A2) and a second reference signal, whereby a second referenced output signal is generated, and
- the first referenced output signal is compared to the second referenced output signal.

7. System according to one of Claims 1 to 4, **characterized in that**
- a first analytical result is derived in the evaluation unit (15) from the first output signal (A1) using first calibration data,
- a second analytical result is derived in the evaluation unit (15) from the second output signal (A2) using second calibration data, and
- the first and second analytical results are compared to obtain the information about interference of the measurement.

8. System according to any one of the preceding claims, **characterized in that** a filter, preferably a bandpass filter (14) is located between the light sensor (5) and the frequency-selective amplifier (10, 11).

9. System according to any one of the preceding claims, **characterized in that** the frequency-selective amplifier (10, 11) is software implemented in an electronic component.

10. System according to Claim 9, **characterized in that** the frequency-selective amplifiers (10, 11) are implemented in the same electronic component.

11. Method for measuring and evaluating photometric signals for detecting an analyte in an analysis liquid, which is applied to an optical evaluation zone (3) of a test carrier (2),
the optical evaluation zone (3) is illuminated by a powered light source (4) and the light remitted from the optical evaluation zone (3) is received by a light sensor (5), processed and converted into a measuring signal, which is fed to a first frequency-selective amplifier (10)
**characterized by** the following steps:
- generating, by a first signal source (6), a first control signal (AN1) having a frequency (F1) and an intensity (I1),
- generating, by a second signal source (7), a second control signal (AN2) having a frequency (F2) and an intensity (12),
- generating a light control signal from the first control signal (AN1) and the second control signal (AN2) for activating the light source (4),
- feeding the measuring signal received from the light sensor (5) to the first frequency-selective amplifier (10) and to a second frequency-selective amplifier (11),
- feeding the first control signal (AN1) to the first frequency-selective amplifier (10) and feeding the second control signal (AN2) to the second frequency-selective amplifier (11),
- feeding a first output signal (A1) of the first frequency-selective amplifier (10) and a second output signal (A2) of the second frequency-selective amplifier (11) to an evaluation unit (15),
- comparing the first output signal (A1) to the second output signal (A2) in the evaluation unit (15), and
- determining information about interference with the measurement by external light from the result of the comparison.

## Revendications

1. Dispositif conçu pour détecter et évaluer des signaux optiques, afin de détecter un analyte dans un liquide d'analyse, comprenant :
- un support de test (2) destiné à recevoir un liquide d'analyse et comportant une zone d'évaluation optique (3),
- une source de lumière (4) pour éclairer la zone d'évaluation optique (3) du support de test (2) ;
- une première source de signal (6) conçue pour produire un premier signal de commande (AN1) pour la source de lumière (4) avec une fréquence F1 et une intensité I1,
- un capteur de lumière (5) conçu pour recevoir, traiter et transformer en un signal de mesure la lumière renvoyée par la zone d'évaluation optique (3) ;
- un premier amplificateur à sélection de fréquence (10) auquel sont amenés le signal de mesure ainsi que le premier signal de commande (AN1) de la première source de signal (6) et à la sortie duquel est délivré un premier signal de sortie (A1),
**caractérisé par**
- une seconde source de signal (7) conçue pour générer un second signal de commande (AN2) avec une fréquence (F2) et une intensité (I2),
- une unité mélangeuse (8) conçue pour produire à partir du premier signal de commande (AN1) et du second signal de commande (AN2) un signal de commande de lumière (2) utilisé pour commander la source de lumière (4),
- un second amplificateur à sélection de fréquence (11) auquel sont amenés le signal de mesure ainsi que le second signal de commande (AN2) de la seconde source de signal (7) et à la sortie duquel est délivré un second signal de sortie (A2),
- une unité d'évaluation (15) à laquelle sont amenés le premier signal de sortie (A1) et le second signal de sortie (A2) et qui est conçue de manière à comparer les deux signaux de sortie (A1, A2) et à déterminer à partir du résultat de la comparaison une information sur une perturbation de la mesure par une lumière parasite.

2. Dispositif selon la revendication 1, **caractérisé par**
- une troisième source de signal pour produire un troisième signal de commande avec une fréquence F3 et une intensité I3,
- le signal de commande de lumière (2) étant produit à partir du premier signal de commande (AN1), du second signal de commande (AN2) et du troisième signal de commande,
- un troisième amplificateur à sélection de fréquence auquel sont amenés le signal de mesure ainsi que le troisième signal de commande de la troisième source de signal et à la sortie duquel est délivré un troisième signal de sortie,
- le troisième signal de sortie étant amené à l'unité d'évaluation, qui compare le premier signal de sortie (A1), le second signal de sortie (A2) et le troisième signal de sortie.

3. Dispositif selon la revendication 1, **caractérisé en ce que** précisément deux amplificateurs à sélection de fréquence (10, 11) sont prévus et qu'en cas d'écart entre les deux signaux de sortie (A1, A2) et un résultat de comparaison théorique, la mesure effectuée est reconnue comme erronée.

4. Dispositif selon une des revendications précédentes, **caractérisé en ce que** les sources de signal (6, 7) sont conçues de sorte que les intensités (I1, I2) du premier et du second signal de commande (AN1, AN2) sont différentes.

5. Dispositif selon une des revendications précédentes, **caractérisé en ce que** la source de signal (6) est conçue de sorte que le premier signal de commande (AN1) est un signal rectangulaire avec une fréquence fondamentale et **en ce que** la source de signal (7) est conçue de sorte que le second signal de commande (AN2) est une harmonique du premier signal de commande (AN1), la fréquence (F2) du second signal de commande (AN2) étant un multiple de la fréquence fondamentale, de préférence le triple de la fréquence fondamentale, et l'intensité (I2) du second signal de commande étant différente de l'intensité (I1) du premier signal de commande.

6. Dispositif selon une des revendications précédentes, **caractérisé en ce que** l'unité d'évaluation (15) est conçue de sorte que
- le premier signal de sortie (A1) est mis en rapport avec un premier signal de référence, produisant de la sorte un premier signal de sortie référencé,
- le second signal de sortie (A2) est mis en rapport avec un second signal de référence, produisant de la sorte un second signal de sortie référencé, et
- le premier signal de sortie référencé est comparé avec le second signal de sortie référencé.

7. Dispositif selon une des revendications 1 à 4, **caractérisé en ce que**
- dans l'unité d'évaluation (15) un premier résultat analytique est déduit du premier signal de sortie (A1) au moyen de premières données de calibrage,
- dans l'unité d'évaluation (15) un second résultat analytique est déduit du second signal de sortie (A2) au moyen de secondes données de calibrage, et
- le premier et le second résultat analytique sont comparés pour obtenir l'information sur une perturbation de la mesure.

8. Dispositif selon une des revendications précédentes, **caractérisé en ce qu'**un filtre, de préférence un filtre passe-bande (14) est disposé entre le capteur de lumière (5) et l'amplificateur à sélection de fréquence (10, 11).

9. Dispositif selon une des revendications précédentes, **caractérisé en ce que** les amplificateurs à sélection de fréquence (10, 11) sont implémentés sous forme de logiciel dans un composant électronique.

10. Dispositif selon la revendication 9, **caractérisé en ce que** les amplificateurs à sélection de fréquence (10, 11) sont implémentés dans un même composant électronique.

11. Procédé pour détecter et évaluer des signaux photométriques, afin de détecter un analyte dans un liquide d'analyse déposé sur une zone de test (3) d'un support de test (2),
dans lequel la zone de test (3) est éclairée par une source de lumière alimentée (4) et la lumière renvoyée par la zone de test (3) est reçue, traitée et convertie par un capteur de lumière (5) en un signal de mesure qui est amené à un premier amplificateur à sélection de fréquence (10), **caractérisé par** les étapes suivantes :
- produire un premier signal de commande (AN1) avec une fréquence F1 et une intensité I1 dans une première source de signal (6),
- produire un second signal de commande (AN2) avec une fréquence F2 et une intensité I2 dans une seconde source de signal (7),
- générer un signal de commande de lumière à partir du premier signal de commande (AN1) et du second signal de commande (AN2) pour commander la source de lumière (4),
- amener le signal de mesure reçu par le capteur de lumière (5) au premier amplificateur à sélection de fréquence (10) et à un second amplificateur à sélection de fréquence (11),
- amener le premier signal de commande (AN1) au premier amplificateur à sélection de fréquence (10) et amener le second signal de commande (AN2) au second amplificateur à sélection de fréquence (11),
- amener un premier signal de sortie (A1) du premier amplificateur à sélection de fréquence (10) et un second signal de sortie (A2) du second amplificateur à sélection de fréquence (11) à une unité d'évaluation (15),
- comparer le premier signal de sortie (A1) avec le second signal de sortie (A2) dans l'unité d'évaluation (15), et
- déterminer à partir du résultat de la comparaison une information sur une perturbation de la mesure par une lumière parasite.
